# EUROPEAN PATENT APPLICATION

(11) **EP 3 054 002 A1**
(43) Date of publication of application: **10.08.2016**
(21) Application number: 13894867.4
(22) Date of filing: 30.09.2013
(51) Int. Cl.: C12M 1/34, G06F 19/10

(54) **COLONY IMAGE INSPECTION PROGRAM, COLONY IMAGE INSPECTION METHOD, AND COLONY IMAGE INSPECTION DEVICE**

(71) Applicant: Fujitsu Limited, Kawasaki-shi, Kanagawa 211-8588 (JP)
(72) Inventor: HAYASHI, Mimiko, Kawasaki-shi Kanagawa 211-8588 (JP); MIYAZAKI, Akira, Fukuoka 814-0001 (JP); MIYAJIMA, Sachiko, Kawasaki-shi Kanagawa 211-8588 (JP); SAGA, Susumu, Kawasaki-shi Kanagawa 211-8588 (JP); KAWANO, Kiyoshi, Kawasaki-shi Kanagawa 211-8588 (JP); HIDAKA, Kouichi, Kawasaki-shi Kanagawa 211-8588 (JP)
(74) Representative: Hoffmann Eitle
(86) International application number: PCT/JP2013/076625
(87) International publication number: WO 2015/045183

(57) **Abstract**

A colony image inspection device (10) acquires each of captured images of three or more petri dishes in each of which the same type of bacterial colony is cultured to be inspected. Furthermore, the colony image inspection device (10) calculates a similarity among the acquired three or more images. Furthermore, the colony image inspection device (10) selects two images indicating that the similarity is the lowest from among the three or more images. Furthermore, the colony image inspection device (10) outputs the selected two images as images that are arranged side by side.

## Description

### [Technical Field]

The embodiments discussed herein are directed to a colony image inspection program, a colony image inspection method, and a colony image inspection device.

### [Background Art]

As an inspection related to food products, in order to check whether the hygiene inspection itself is accurately performed, the inspection quality is sometimes investigated other than a hygiene inspection that inspects whether fungi are mixed. In the investigation of the inspection quality, for example, in order to confirm whether there is no problem with an inspection procedure, an inspection apparatus, and reagents of the inspection performed in an inspection room every day, in some cases, a specimen for investigation is selected and inspection quality check is performed for each certain period of time or for each certain number of inspections. Furthermore, in a daily hygiene inspection work, the inspection quality may also sometimes be confirmed. However, the current hygiene inspection work only manages the number of colonies visually counted or counted by an apparatus and determines, by using only a numerical value, whether the inspection criteria is satisfied. However, even if the numerical value is within a reference value, an abnormal pattern, such as a case in which a fungus that is not supposed to be present in the target foodstuff is grown, a case in which an object other than fungi (molds, etc.) that are primarily wanted to be counted is bred, a case in which an unusual breeding state is present, a case of contamination with a foreign substance or damage of a medium, or the like, that does not appear from the a numerical value is present. Determining the growing condition of such fungus is similarly limited to inspectors who have the know-how.

### [Citation List]

### [Patent Literature]

Patent document 1: Japanese Laid-open Patent Publication No. 62-247250
Patent document 2: Japanese Laid-open Patent Publication No. 2002-24799

### [Summary of invention]

### [Technical Problem]

However, it takes a lot of time and effort to investigate an abnormality of the inspection or the inspection quality other than a numerical value in a daily hygiene inspection work.

Namely, in the hygiene inspection described above, an immense number of petri dishes in which colonies are cultured are created. Consequently, the time taken to check all of the petri dishes and taken to specify a petri dish in which an abnormality occurs in the course of the culture and the efforts thereof are enormous. Furthermore, it is difficult to investigate the inspection quality without the know-how that can determine an abnormality on the basis of a food product, the type of fungi possibly be grown in the subject food product, a breeding state, or the like. The personnel having the subject know-how is limited and it is difficult to sufficiently grant such personnel the time to investigate the inspection quality. Thus, even for a contact person who does not have the know-how, by collecting images by capturing the images of the enormous number of the petri dishes in a daily hygiene inspection work; by determining the images in a system; and by the inspectors who have the know-how checking only the selected images, whereby the number of steps to be performed by the inspector can be reduced and thus the inspection quality can be checked within the daily hygiene inspection work.

Accordingly, it is an object in one aspect of an embodiment of the invention to provide a colony image inspection program, a colony image inspection method, and a colony image inspection device that can reduce the trouble of investigating inspection quality.

### [Solution to Problem]

A colony image inspection program according to an aspect of an embodiment causes a computer to execute a process of acquiring each of captured images of three or more petri dishes in each of which the same type of bacterial colony is cultured to be inspected. Furthermore, the colony image inspection program causes the computer to execute a process of calculating a similarity among the acquired three or more images. Furthermore, the colony image inspection program causes the computer to execute a process of selecting two images that indicates that the similarity is the lowest from among the three or more images. Furthermore, the colony image inspection program causes the computer to execute a process of outputting the selected two images as images that are arranged side by side.

### [Advantageous Effects of Invention]

It is possible to reduce the trouble of investigating inspection quality.

### [Brief Description of Drawings]

FIG. 1 is a schematic diagram illustrating the configuration of a quality management system according to a first embodiment.
FIG. 2 is a block diagram illustrating the functional configuration of a colony image inspection device according to the first embodiment.
FIG. 3 is a schematic diagram illustrating an example of inspection information.
FIG. 4 is a schematic diagram illustrating an example of a condition specifying screen.
FIG. 5 is a schematic diagram illustrating an example of the condition specifying screen.
FIG. 6 is a schematic diagram illustrating an example of a method of calculating the similarity.
FIG. 7 is a schematic diagram illustrating an example of an inspection quality investigation screen.
FIG. 8 is a schematic diagram illustrating an example of the inspection quality investigation screen.
FIG. 9 is a schematic diagram illustrating an example of an image list screen.
FIG. 10 is a flowchart illustrating the flow of a colony image inspection process according to the first embodiment.
FIG. 11 is a schematic diagram illustrating an example of a computer that executes a colony image inspection program according to each of the first embodiment and a second embodiment.

### [Embodiments for Carrying Out the Invention]

Preferred embodiments of a colony image inspection program, a colony image inspection method, and a colony image inspection device disclosed in the present invention will be described in detail below with reference to the accompanying drawings. The present invention is not limited to the embodiments. Each of the embodiments can be appropriately used in combination as long as processes do not conflict with each other.

### First Embodiment

### [System configuration]

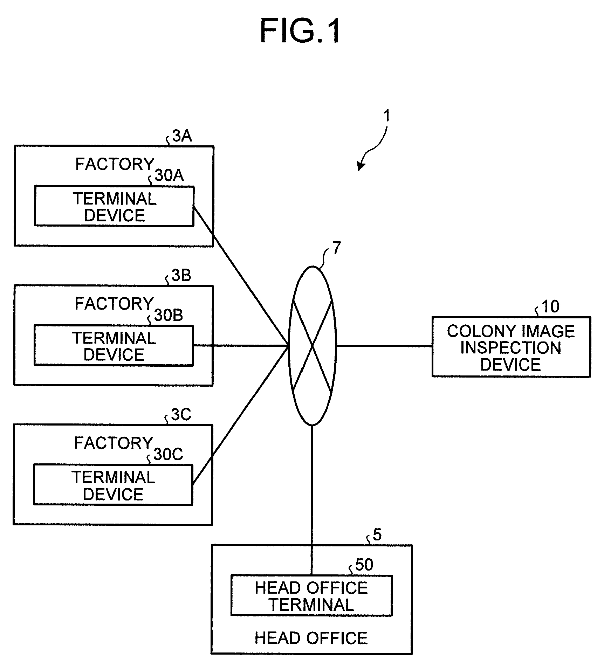

FIG. 1 is a schematic diagram illustrating the configuration of a quality management system according to a first embodiment. A quality management system 1 illustrated in FIG. 1 supports quality management of a hygiene inspection that is performed on the food products shipped from factories 3A to 3C. As a part of the quality management, when the quality of a hygiene inspection is to be investigated by checking an image of a colony that was used for the hygiene inspection in each of the factories 3A to 3C, the quality management system 1 provides a colony image inspection service that can reduce the number of images of the colonies to be checked. Furthermore, in a description below, as only an example, a description will be given by exemplifying a case in which the quality of hygiene inspection that is performed in an inspection department in each of the factories 3A to 3C is investigated by a quality management department in a head office 5; however, the applied case is not limited to this example.

As illustrated in FIG. 1, in the quality management system 1, a colony image inspection device 10, terminal devices 30A to 30C, and a head office terminal 50 are accommodated. In FIG. 1, three terminal devices and a single head office terminal are illustrated; however, the number of each of the devices accommodated in the system is not limited to the configuration illustrated in the drawing. Namely, the quality management system 1 can accommodate an arbitrary number of terminal devices and head office terminals. Furthermore, in a description below, there may be a case in which the base locations of the respective factories 3A to 3C are collectively referred to as a "factory 3" and a case in which the devices of the terminal devices 30A to 30C are collectively referred to as a "terminal device 30".

The colony image inspection device 10, the terminal devices 30, and the head office terminal 50 are connected via a network 7 such that they can communicate with each other. Any kind of communication network, such as the Internet, a local area network (LAN), a virtual private network (VPN), or the like, may be used as the network 7 irrespective of whether the network is a wired or wireless connection.

From among these, the colony image inspection device 10 is a computer that provides the colony image inspection service described above for the terminal devices 30 and the head office terminal 50. For example, the colony image inspection device 10 may also be mounted as a Web server that implements the colony image inspection service or the colony image inspection device 10 can also be mounted as cloud computing that provides an outsourced colony image inspection service. For another example, the colony image inspection device 10 can also be mounted by preinstalling or installing, in a desired computer, a colony image inspection program that is provided as packaged software or online software. In any of the cases of implementation, the colony image inspection device 10 does not always need to be implemented as an external device of the terminal devices 30 or the head office terminal 50 and may also provide one of the terminal devices 30 and the head office terminal 50 with a colony image inspection service.

The terminal device 30 is a computer to which an imaging device that is an apparatus that can capture an image of a petri dish that is used for hygiene inspection by a person involved in an inspection department in the factory 3 is connected or a computer that can store captured images of petri dishes by receiving the images from the imaging device via wireless or wired communication. Furthermore, the captured images of the petri dishes may also be acquired from one of removable media that can be connected to the computer. Examples of the terminal device 30 that can be used here include, in addition to a fixed terminal, such as a personal computer, a mobile communication terminal, such as a smart phone, a mobile phone device, a PHS, a PDA, or the like, and, furthermore, a tablet terminal or the like that does not have a function of connecting to a mobile telecommunications network. Furthermore, the PHS described above is an abbreviation of a "Personal Handyphone System" and the PDA described above is an abbreviation of a "Personal Digital Assistants".

The head office terminal 50 is a computer that is used by a person involved in a quality management department in the head office 5. Examples of the head office terminal 50 that can be used here include, in addition to a fixed terminal, such as a personal computer, a mobile communication terminal, such as a smart phone, a mobile phone device, a PHS, a PDA, or the like, and, furthermore, a tablet terminal or the like that does not have a function of connecting to a mobile telecommunication network. Furthermore, a description will be given here with the assumption that the colony image inspection service is provided, via the head office terminal 50, to a person involved in the quality management department in the head office 5; however, the colony image inspection service may also be provided via the terminal devices 30 used by the persons involved in the inspection department in each of the factories 3, for example, the responsible person in the inspection department or the person involved conforming to this. In this case, it is possible to allow the responsible person in the factory 3 or the person involved to browse by narrowing down to the hygiene inspection related to each of the factories 3.

In the following, an example of the hygiene inspection performed in the factory 3 will be described. For example, in order to perform a hygiene inspection on the food products that are shipped from the factory 3, an inspector belonging to the inspection department in the factory 3 collects a part of the food products as a specimen and cultures a colony of bacteria in a petri dish in the following method.

For example, in the hygiene inspection, a part of the food products is collected by an inspector as a specimen and then the identification information, for example, a specimen number numbered for each specimen is attached to the collected specimen. Then, for example, in the pour plate method, in accordance with the various kinds of inspection items, such as Staphylococcus aureus, Escherichia coli, general viable bacteria(Aerobic colony count), or the like, the inspector puts the specimen in a petri dish together with a melted medium. At this time, it is possible to create a plurality of petri dishes in each of which the same specimen is diluted at the same dilution step. Furthermore, for the petri dishes in each of which the same specimen is cultured, the same specimen number is written on the lid of the petri dishes such that the inspection item that is supposed to be performed between the same specimens can be specified. Then, the inspector cultures bacteria in petri dishes in an incubator for a predetermined period of time, for example, for about 48 hours in a case of general viable bacteria, that is associated with the inspection item. Furthermore, regarding a specimen collected from the food products, some of the specimen can be cryopreserved in preparation for a re-inspection.

After the culturing, the inspector performs the hygiene inspection by counting the number of colonies in each of the petri dishes. At this time, it is possible to allow the inspector to inspect an actual petri dish; however, it is also possible to allow the inspector to inspect an image of a bacterial colony in a petri dish captured by a predetermined imaging device. In the following, an image of a bacterial colony captured by an imaging device is sometimes referred to as a "colony image".

As an example of the imaging device described above, a colony counter may be used. Some colony counters can acquire colony images, in each of which colonies and media are clearly distinguished, by capturing an image while changing the angle and the direction of the colonies cultured in a petri dish and, furthermore, changing the type of the illumination to be illuminated onto the petri dish. With the colony images, even if an inspector who does not have a sufficient work experience of the hygiene inspection can accurately count the number of colonies and it is also possible to suppress a state in which a difference occurs between the number of colonies in the same petri dish counted by each inspector. Furthermore, the colony counter can perform image processing that automatically counts the number of colonies from a colony image. In this way, when the number of colonies is automatically counted, because an inspector can sometimes determine whether a shipment is OK or NG by just checking whether the number of colonies is within a certain range, the work load of the hygiene inspection can be reduced. Furthermore, it goes without saying that, when the number of colonies is checked, by displaying a colony image together with the number of colonies, it is possible to check whether an obvious error is present in a count performed by the colony counter.

In an environment of the hygiene inspection performed in this way, the terminal device 30 uploads various kinds of information used for the hygiene inspection performed in the factory 3 to the colony image inspection device 10. For example, the terminal device 30 uploads, to the colony image inspection device 10 together with the colony image captured by the colony counter or the like, the information related to the hygiene inspection that was performed by using the subject colony image. Furthermore, in the following, the information related to the hygiene inspection is sometimes referred to as "inspection information".

Furthermore, in the above, a case of performing, in addition to capturing of the colony image performed by the colony counter, automatically counting the number of colonies is exemplified; however, automatically counting the number of colonies may also be performed by the colony image inspection device 10. Furthermore, FIG. 1 exemplifies a case in which the inspection department is present in the factory 3; however, the inspection department does not always need to be present in the factory 3. Alternatively, food products or specimens collected from the food products may also be transported to outside the factory 3.

### [Configuration of the colony image inspection device 10]

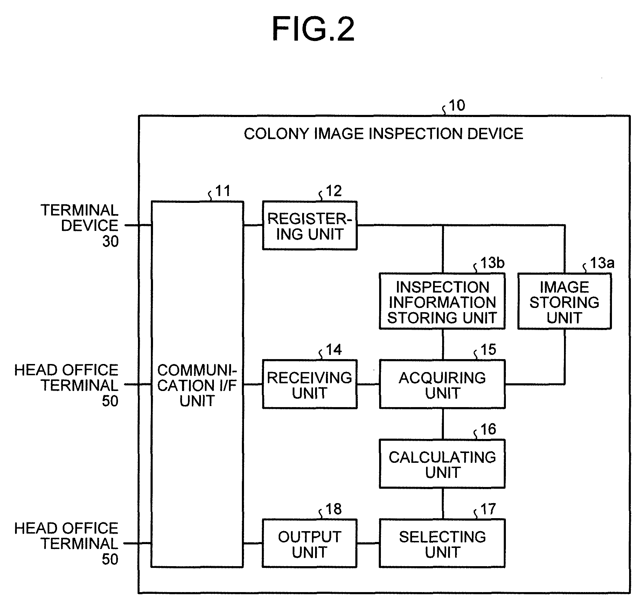

FIG. 2 is a block diagram illustrating the functional configuration of the colony image inspection device 10 according to the first embodiment. As illustrated in FIG. 2, the colony image inspection device 10 includes a communication interface (I/F) unit 11, a registering unit 12, an image storing unit 13a, an inspection information storing unit 13b, a receiving unit 14, an acquiring unit 15, a calculating unit 16, a selecting unit 17, and an output unit 18. Furthermore, in addition to the functioning units illustrated in FIG. 2, the colony image inspection device 10 may also include various kinds of functioning units included in an already known computer, for example, functioning units, such as various kinds of input/output devices, an audio output device, an imaging device, and the like.

From among the functioning units, the communication I/F unit 11 is an interface that performs communication control between other devices, for example, the terminal device 30 and the head office terminal 50. An example of the communication I/F unit 11 that can be used here includes a network interface card, such as a LAN card or the like. For example, the communication I/F unit 11 sends a colony image or an instruction to transmit the inspection information of the colony image to the terminal device 30 or receives the colony image or the inspection information from the terminal device 30. Furthermore, the communication I/F unit 11 receives an investigation request for the inspection quality from the head office terminal 50 or sends an inspection quality investigation screen including a colony image selected for investigating the inspection quality.

The registering unit 12 is a processing unit that registers various kinds of information used for a hygiene inspection in the factory 3 in a predetermined destination to save. For example, the registering unit 12 can acquire a colony image and inspection information by instructing the terminal device 30 to upload at an interval of certain period of time, for example, daily, weekly, or monthly. For another example, the registering unit 12 may also upload, to the terminal device 30 in real time, the colony image and the inspection information that are used for the subject hygiene inspection every time the colony image and the inspection information are registered in the terminal device 30 or every time the hygiene inspection is ended. When the upload is performed in this way, the registering unit 12 registers the colony image from among the pieces of the uploaded information in the image storing unit 13a and registers the inspection information in the inspection information storing unit 13b.

The image storing unit 13a is a storing unit that stores therein colony images. For example, in the image storing unit 13a, the colony images uploaded from the terminal device 30 are registered in the registering unit 12. Furthermore, the colony images may be any formats, such as a graphic interchange format (GIF) file, a joint photographic experts group (JPEG) file, a bit map (BMP) file, or the like.

The inspection information storing unit 13b is a storing unit that stores therein inspection information. An example of the inspection information that can be used here includes data associated with the items, such as a specimen number, a foodstuff, the inspection date, the production/ship date, the ship source, the Lot No, a contact person, the dilution rate, the number of colonies, the storage destination of a colony image, and the like. The "specimen number" mentioned here indicates a specimen number of a specimen and the "foodstuff" indicates the foodstuff to which the food products to which a specimen is collected belongs. Furthermore, the "inspection date" indicates the date on which a hygiene inspection is performed. Here, the date has been exemplified; however, hours, minutes, and seconds may also be additionally attached. Furthermore, the "ship source" indicates a facility that manufactures food products or performs secondary processing on food products and that ships the food products, and, in the example illustrated in FIG. 1, the factory 3A, the factory 3B, and the factory 3C correspond to the "ship sources". Furthermore, the "Lot No" indicates the number that is used to identify a Lot of the food product from which a specimen is collected. Furthermore, the "contact person" indicates an inspector who is in charge of a hygiene inspection. Furthermore, the "number of colonies" indicates the number of colonies that are cultured in a petri dish. For example, even for the same specimen, it is possible to store the number of colonies in a petri dish created for each inspection item, such as Staphylococcus aureus, Escherichia coli, general viable bacteria, or the like. Furthermore, it is also possible to store the number of colonies for plurality of petri dishes in each of which a specimen with the same stage is diluted and cultured related to the same inspection item, i.e., store the number of colonies for plurality of petri dishes in each of which the same specimen is cultured under the same condition. Furthermore, the "storage destination" indicates the storage destination in which a colony image is stored and, for example, a path for the storage destination that stores therein a file of the colony image may be used. Furthermore, FIG. 2 exemplifies a case in which the colony image and the inspection information are individually stored; however, the colony images may also be managed by including the images in the inspection information as binary format data.

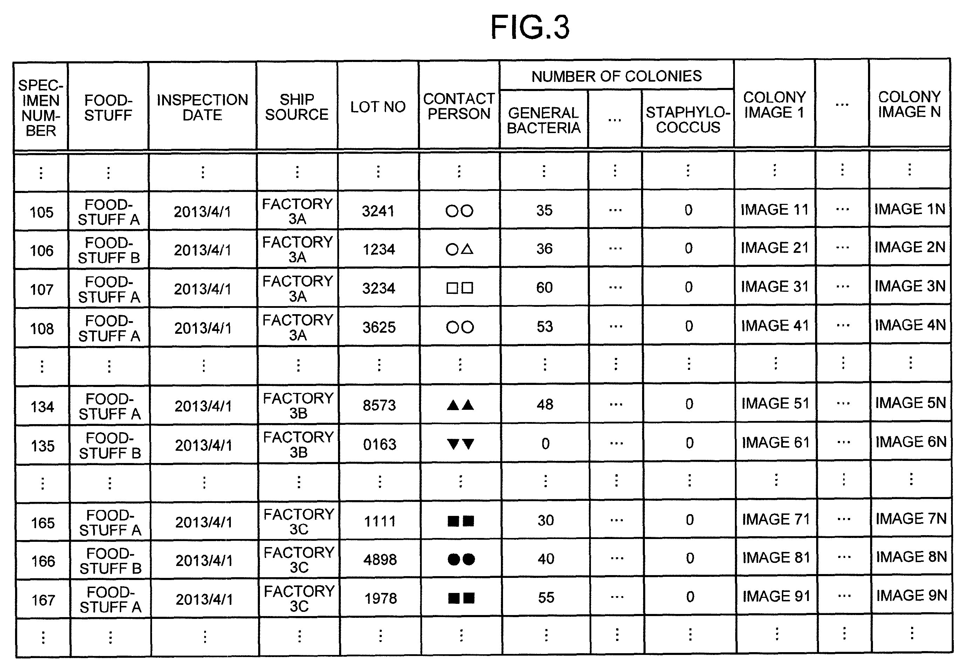

FIG. 3 is a schematic diagram illustrating an example of inspection information. FIG. 3 illustrates an example in which the inspection information related to the hygiene inspection performed on April 1, 2013 is extracted; however, in practice, it is assumed that the inspection information before April 1, 2013 and after April 1, 2013 is stored. Furthermore, in FIG. 3, the inspection information related to the hygiene inspection performed in each of the factories 3A to 3C is illustrated; however, inspection information related to the hygiene inspection performed in another factory may also be further included and the inspection information may also be managed for each factory.

From among the records on the inspection information illustrated in FIG. 3, the records with the specimen numbers of "105" to "108" are related to the hygiene inspections performed in the factory 3A and the records with the specimen numbers of "134" to "135" are related to the hygiene inspections performed in the factory 3B. Furthermore, the records with the specimen numbers of "165" to "167" are related to the hygiene inspections performed in the factory 3C. Regarding these records, a plurality of foodstuffs is targeted for the hygiene inspection and inspections of general viable bacteria, Escherichia coli, Staphylococcus aureus, and the like have been performed. Furthermore, the storage destination of a file is associated with each of the records such that a colony image of general viable bacteria that is cultured in a petri dish, a colony image of Escherichia coli that is cultured in a petri dish, and a colony image of Staphylococcus aureus that is cultured in a petri dish can be called.

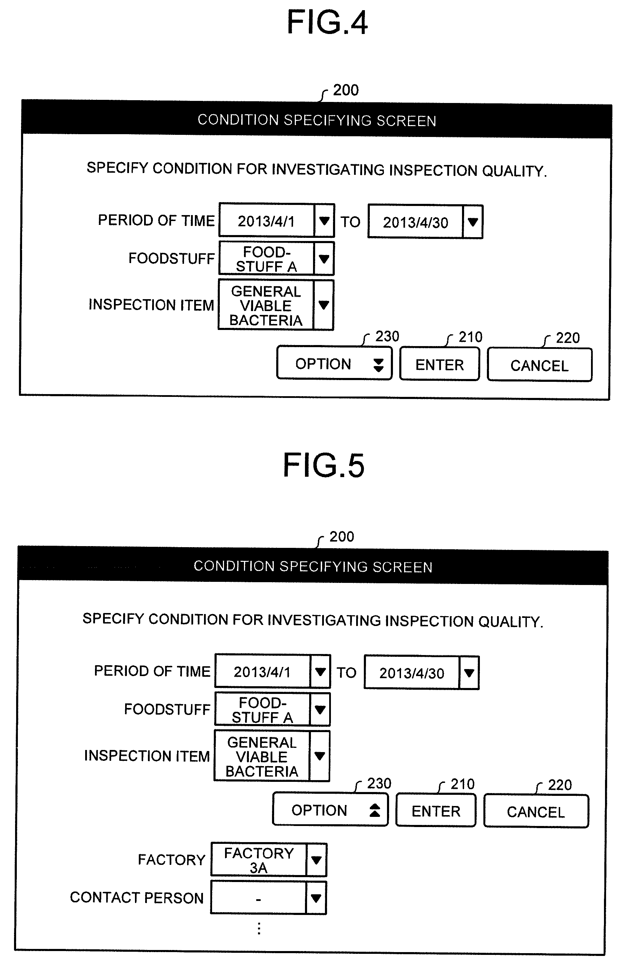

A description will be given here by referring back to FIG. 2. The receiving unit 14 is a processing unit that receives an instruction related to various kinds of conditions. For example, when the receiving unit 14 receives an inspection request for the inspection quality from the head office terminal 50, the receiving unit 14 accepts an instruction of the condition for the investigation of the inspection quality. For example, the receiving unit 14 accepts the inspection request for the inspection quality via the screen, for example, a menu screen including the item, such as "investigation of the inspection quality" or the like, that is provided by a colony image inspection program running on a processor of the colony image inspection device 10. Then, the receiving unit 14 sends the condition specifying screen illustrated in FIG. 4 to the head office terminal 50.

FIG. 4 is a schematic diagram illustrating an example of a condition specifying screen. A condition specifying screen 200 illustrated in FIG. 4 includes a Graphical User Interface (GUI) with a pull-down menu for specifying the condition, such as the "period of time", a "foodstuff", the "inspection item", and the like. From among these, in the pull-down menu of the "period of time", it is possible to specify which period of time of the hygiene inspection is used as the investigation target for the inspection quality from among the hygiene inspections that were performed in the past. Furthermore, in the pull-down menu of the "foodstuff", it is possible to specify, from among the food products, which foodstuff that has been subjected to the hygiene inspection is used as the investigation target for the inspection quality. Furthermore, in the pull-down menu of the "inspection item", it is possible to specify which inspection item from among the inspection items, for example, general viable bacteria, Escherichia coli, and Staphylococcus aureus, is used as the investigation target for the inspection quality. An enter button 210 is pressed and operated in a state in which each of the items is specified by the pull-down menu, whereby the specifying of the "period of time", the "foodstuff", and the "inspection item" is sent from the head office terminal 50 to the colony image inspection device 10.

By doing so, the receiving unit 14 can receive the specifying of the "period of time", the "foodstuff", and the "inspection item" or can receive the specifying of a combination of these. Furthermore, when a cancel button 220 on the condition specifying screen 200 is pressed and operated, the investigation of the inspection quality is stopped.

Furthermore, when an option button 230 on the condition specifying screen 200 is pressed, as illustrated in FIG. 5, the pull-down menu that specifies the conditions of the "factory" and the "contact person". FIG. 5 is a schematic diagram illustrating an example of the condition specifying screen. In the pull-down menu of the "factory" illustrated in FIG. 5, it is possible to specify which hygiene inspection performed in the factory from among the factories that ship food products is used as the investigation target. Furthermore, in the pull-down menu of the "contact person", it is possible to specify, from among all of the inspectors, which of the hygiene inspections performed by which of the inspector who is in charge is used as the investigation target for the inspection quality. The enter button 210 is pressed and operated in a state in which each of the items is specified by the pull-down menu, whereby the specifying of the "factory" and the "contact person" is sent from the head office terminal 50 to the colony image inspection device 10. Furthermore, here, a case in which a single condition can be specified for each item is exemplified; however, a plurality of conditions may also be specified for each single item. For example, a plurality of pull-down menus for specifying a foodstuff, a factory, and a contact person may also be provided on the condition specifying screen 200.

Consequently, the receiving unit 14 can receive the specifying of the "factory" and the "contact person" or receive a combination thereof.

Furthermore, on the condition specifying screen 200 described above, a case in which the conditions can be specified related to the five items, i.e., the "period of time", the "foodstuff", the "inspection item", the "factory", and the "contact person"; however, all the conditions of the five items do not always need to be specified. For example, for the item in which no condition is specified, a blank or "not specified" may be selected. Furthermore, FIG. 4 and FIG. 5 each illustrate a case in which the various kinds of conditions are specified by using the pull-down menus; however, any method may also be used as long as an interface that can input or select a condition is used.

A description will be given here by referring back to FIG. 2. The acquiring unit 15 is a processing unit that acquires a colony image of each of three or more petri dishes in which the same type of bacterial colony is included.

For example, the acquiring unit 15 searches for the inspection information stored in the inspection information storing unit 13b in accordance with the specification of the condition received by the receiving unit 14. For example, in the example of the condition specifying screen illustrated in FIG. 4, the period of time "April 1, 2013" to "April 30, 2013" is specified as the condition, a foodstuff A is specified as the condition, the inspection item of "general viable bacteria" is specified as the condition. In this case, the inspection information illustrated in FIG. 3 is searched by using the period of time and the foodstuff described above for an AND condition. Consequently, the records of the specimen numbers "105", "107", "108", "134", "165", and "167" are searched from among the records of the inspection information illustrated in FIG. 3. Furthermore, in FIG. 3, because the record of the inspection information on April 1, 2013 is extracted, an example in which the six records described above are narrowed down is illustrated; however, in practice, if a record that matches the condition described above is present from among the records between April 2 to April 30, 2013, the matched record is also searched. Then, the acquiring unit 15 refers to the storage destination of the colony image associated with the inspection item of "general viable bacteria" from among the storage destinations of the colony images included in the searched record and acquires a colony image of cultured general viable bacteria from the image storing unit 13a. Consequently, it is possible to narrow down the inspection of the general viable bacteria in the foodstuff A performed in April to the investigation target.

Furthermore, as illustrated in FIG. 5, if the factory "factory 3A" is further specified as the condition, in addition to the period of time and the foodstuff described above, the factory is used as the AND condition and the inspection information illustrated in FIG. 3 is searched. In this case, from among the records of the inspection information illustrated in FIG. 3, the records with the specimen numbers of "105", "107", and "108" are searched. Furthermore, in FIG. 3, because the record of the inspection information on April 1, 2013 has been extracted, an example in which only the three records described above are narrowed down is illustrated; however, in practice, if records that satisfy the condition described above are present in the records from April 2 to 30, 2013, the subject records are also searched. Then, the acquiring unit 15 refers to the storage destination of the colony image associated with the inspection item of "general viable bacteria" from among the storage destinations of the colony images included in the previously searched records and acquires the colony image of cultured general viable bacteria from the image storing unit 13a. Consequently, it is possible to narrow down the inspection of the general viable bacteria in the foodstuff A performed in the factory 3A in April to the investigation target.

Furthermore, on the condition specifying screen 200 illustrated in FIG. 5, it is assumed of a case in which, instead of the factory of "factory 3A", a contact person "OO" is specified as the condition. In this case, in addition to the period of time and the foodstuff described above, a contact person is used as an AND condition and then the inspection information illustrated in FIG. 3 is searched. Consequently, from among the records of the inspection information illustrated in FIG. 3, the records with the specimen numbers "105" and "108" are searched. Furthermore, in FIG. 3, because the record of the inspection information on April 1, 2013 is extracted, an example in which only the two records described above are extracted is illustrated; however, in practice, if records that satisfy the condition described above is present in the records from April 2 to 30, 2013, the subject records are also searched. Then, the acquiring unit 15 refers to the storage destination of the colony image associated with the inspection item of "general viable bacteria" from among the storage destinations of the colony images included in the previously searched records and acquires the colony image of cultured general viable bacteria from the image storing unit 13a. Consequently, it is possible to narrow down the inspection of the general viable bacteria in the foodstuff A performed by the inspector indicated by "OO" in April to the investigation target.

Furthermore, here, a case in which AND search is performed on the items in each of which a condition is specified is exemplified; however, an OR search may also be performed on the items in each of which a condition is specified. Furthermore, it may also possible to allow a person involved in the quality management department in the head office 5 to select whether the AND search is to be performed or the OR search is to be performed.

The calculating unit 16 is a processing unit that calculates the similarity among three or more colony images acquired by the acquiring unit 15.

For example, the calculating unit 16 can use a self-organizing map (SOM) method for calculating the similarity between the colony images. Specifically, the calculating unit 16 sets, as the evaluation axis for evaluating a bacteria image, the color of a colony, the size, the number of the colonies, the degree of circularity, the degree of gross, the distribution in a petri dish, and the like. Then, by performing an image process on each of the colony images acquired by the acquiring unit 15, the calculating unit 16 creates, for each colony image, high-dimensional vector data related to the color of a colony, the size, the number of the colonies, the degree of circularity, the degree of gross, and the distribution in a petri dish. Then, the calculating unit 16 maps the high-dimensional vector data on each of the colony images onto a low dimensional map space with about one to three-dimensional space. Then, the calculating unit 16 calculates the distance between each of the colony images. Consequently, calculation of the similarity can be implemented without teacher data. Furthermore, the item with the evaluation axis may also be set as a default by a producer of the colony image inspection program or the like or a person involved in the quality management department in the head office 5 may also be selected each time.

For another example, the calculating unit 16 may also extract a feature value from a colony image by using a predetermined algorithm and calculate the similarity from the feature value between the colony images. For the extraction of such a feature value, as an example, an arbitrary algorithm, such as the Scale-Invariant Feature Transform (SIFT) algorithm, the Speed Up Robust Features (SURF) algorithm, or the Histogram of Oriented Gradients (HOG) algorithm, that obtain feature values from the magnitude or the direction of the brightness gradient may be used. In this case, a feature value is converted to vector data and then the similarity of the feature vector between colony images can be calculated. By using the algorithms, it is possible to extract a feature value that is robust against the rotation or scale transformation of an image. For another example, it is also possible to extract a color histogram of a colony image as a feature value. In this way, when a color histogram is created, for example, a plurality of wavelength components, for example, each of the color components of the red component R, the green component G, and the blue component B, is reduced to four colors; a combination of the red component R, the green component G, and the blue component B is set to 64 patterns; and bin numbers of 0 to 63 are attached to the respective colors with 64 patterns. Then, by counting the number of pixels that correspond to the colors with the respective bin numbers in the colony image, a color histogram can be created. Then, by comparing the shape or the like of the color histogram between the colony images, the similarity can be calculated. For another example, it is also possible to extract the average value of the colony areas included in a colony image as a feature value. In this way, when the average value of the colony areas is obtained, by performing image processing, such as edge detection or the like, on a colony image, the colony with a closed edge is detected. Then, by summing up the number of pixels of each of the colonies, the total area of the colonies is obtained. Furthermore, by dividing the total area of the colonies by the number of colonies, the average value of the colony areas is obtained. Then, for example, in a pair of colony images that are used to calculate the similarity, between the average value of the colony areas, by dividing a smaller value by a greater value, it is possible to calculate the similarity closer to 1 as the two images are similar.

In this way, when the similarity is calculated by extracting the feature value from each of the colony images, the colony image that is used to calculate the similarity can be paired. For example, the calculating unit 16 sets a single image from among the colony images acquired by the acquiring unit 15 as a reference image and can calculate the similarity between the reference image and each of the colony images other than the reference image. In this way, when a reference image is selected from the colony images, an image from among all of the colony images can be randomly selected. Furthermore, it is also possible to allow a person involved in the quality management department in the head office 5 to browse the colony images by a thumbnail display or the like and to specify the colony image in which the formation of the colony is typical in view of the type of the foodstuff from which the inspection quality is investigated. Furthermore, the calculating unit 16 can also calculate, in a round robin manner, the similarity of all the combination of the colony images acquired by the acquiring unit 15.

For another example, the calculating unit 16 can also calculate the similarity after converting a colony image to a spatial frequency area. Here, a case of using the Fourier transformation has been exemplified; however, another conversion method may be used as long as the algorithm that can convert to a spatial frequency area is used. For example, Discrete Fourier Transform (DFT), Fast Fourier Transform (FFT), Discrete Cosine Transform (DCT), or the like may be used.

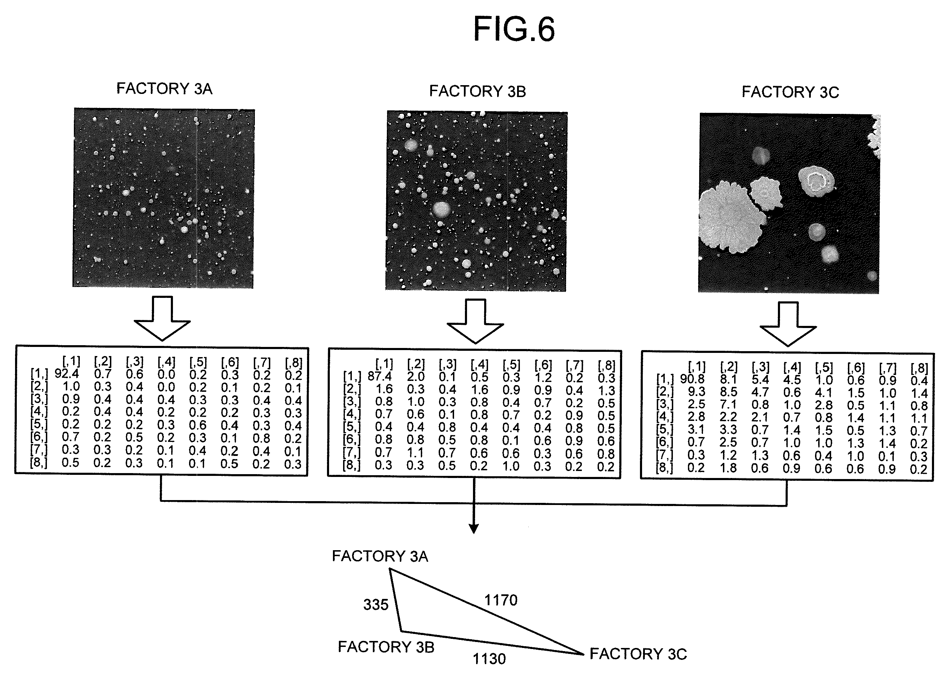

FIG. 6 is a schematic diagram illustrating an example of a method of calculating the similarity. FIG. 6 illustrates the colony image of the factory 3A, the colony image of the factory 3B, and the colony image of the factory 3C that are used for the hygiene inspection of general viable bacteria in the order from the left. For the three colony images illustrated in FIG. 6, binarization has already been performed and, on each of the lower portions thereof, the coefficient matrix that is the result of the Fourier transformation performed on each of the colony images is illustrated. These coefficient matrices indicate that the image contains greater number of low-frequency components as the coefficient located at the upper left is greater and indicates that the image contains the greater number of high-frequency components as the coefficient located at the lower right is greater. When the distance, for example, the Euclidean distance, of the coefficient matrix between the colony images, the following result is obtained. Namely, the distance between the colony image of the factory 3A and the colony image of the factory 3B is "335". Furthermore, the distance between the colony image of the factory 3B and the colony image of the factory 3C is "1130". Furthermore, the distance between the colony image of the factory 3C and the colony image of the factory 3A is "1170". Namely, this example indicates that the colony image of the factory 3A and the colony image of the factory 3B are the most similar and indicates that the colony image of the factory 3C and the colony image of the factory 3A are the most dissimilar. Because these distances indicate that as the value of the distance is smaller the degree of similarity each other is high, the distances can be used as an example of the similarity without processing anything or by normalizing the distances.

Furthermore, here, it is assumed of a case in which one of the methods of calculating the similarity described above is selectively used; however, statistical values of the similarity calculated by each of the calculation method, for example, arithmetic mean values, weighted average values, mode values, median values, or the like, may also be used as the overall similarity. Furthermore, from among the methods of calculating the similarity described above, for the feature values extracted from an image, for example, the feature values that are used to calculate the similarity by using a conversion to color histograms or spatial frequency components, the feature value can be calculated in advance. By doing so, because the similarity can be calculated by using the previously calculated feature value, a process response can be improved.

The selecting unit 17 is a processing unit that selects two colony images that indicate the similarity is the most dissimilar from among three or more colony images. For example, if the similarity that indicates two colony images are similar as a value is greater, for example, if the correlation coefficients, the normalized distances, or the like is used by the calculating unit 16, the selecting unit 17 selects the two colony images with the lowest similarity from among the colony images acquired by the acquiring unit 15. Furthermore, if the similarity that indicates two colony images are similar as a value is smaller indicated by the calculating unit 16, for example, if the distance, such as the Euclidean distance or the like, is used by the calculating unit 16, the selecting unit 17 selects the two colony images having the greatest similarity from among the colony images acquired by the acquiring unit 15.

For example, if the self-organizing map (SOM) method is used, the selecting unit 17 selects the pair of colony images, from among the colony images mapped onto the low dimensional map space, having the greatest distance, i.e., the pair of colony images that are the most dissimilar. Furthermore, if the similarity between the reference image and the colony image other than the reference image is obtained, the selecting unit 17 selects, as a pair, the colony image in which the similarity between the reference image is the lowest and the reference image. Furthermore, if the similarity between each of the colony images is obtained in a round-robin manner, the selecting unit 17 selects a pair of colony images that has the lowest similarity from among all of the combinations of the colony images. Furthermore, if the similarity is obtained by converting the colony images to the spatial frequency area, the selecting unit 17 selects a pair of colony images with the greatest distance from among all of the combinations of the colony images, in other words, a pair colony images that are the most dissimilar each other.

The output unit 18 is a processing unit that outputs two colony images selected by the selecting unit 17 as images that are arranged side by side. For example, the output unit 18 creates an inspection quality investigation screen in which the most two dissimilar colony images are vertically or horizontally arranged and sends the created inspection quality investigation screen to the head office terminal 50.

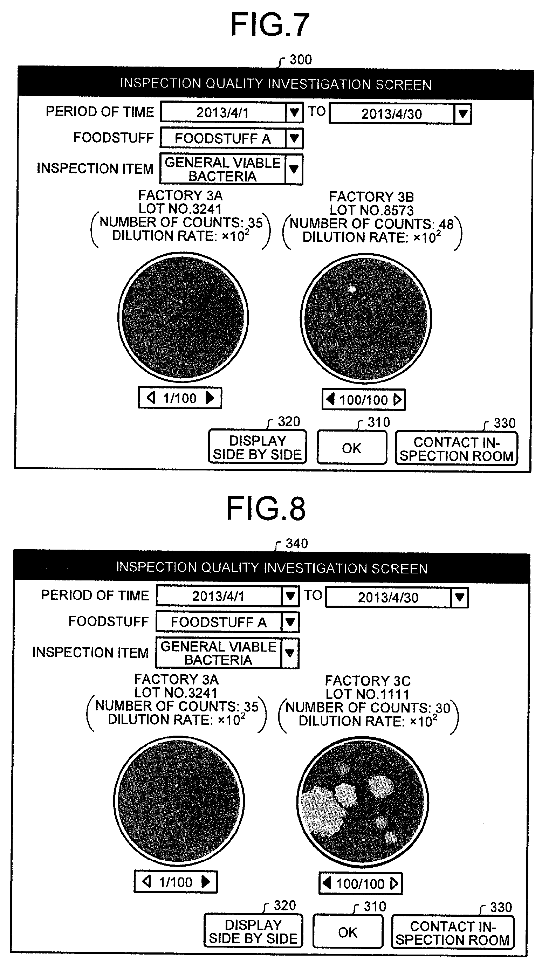

FIG. 7 and FIG. 8 are schematic diagrams each illustrating an example of an inspection quality investigation screen. FIG. 7 and FIG. 8 illustrates a display example of a case in which the colony images are acquired in accordance with the condition specified by the condition specifying screen 200 illustrated in FIG. 4. Between these, in FIG. 7, from among the colony images associated with the inspection information illustrated in FIG. 3, the colony image with the Lot No. of 3241 of the factory 3A and the colony image with the Lot No. of 8573 of the factory 3B are illustrated as the pair of the most dissimilar colony images. Furthermore, in FIG. 8, from among the colony images associated with the inspection information illustrated in FIG. 3, the colony image with the Lot No. of 3241 of the factory 3A and the colony image with the Lot No. of 1111 of the factory 3C are illustrated as the pair of the most dissimilar colony image. Furthermore, FIG. 7 and FIG. 8 illustrate a case in which, as an example of the information related to the colony image, the number of counts, the dilution rate, and the like are displayed; however, another information, for example, the inspection date, a contact person, or the like may also be displayed.

For example, if an inspection quality investigation screen 300 illustrated in FIG. 7 is displayed, as the pair of colony images that are the most dissimilar from among the large number of colony images, even if a slight difference is present in the number of colonies, the distribution mode of the colonies, the magnitude of the granularity of the colonies, the images that are much the same are arranged side by side. In this case, the person involved in the quality management department in the head office 5 can determine that, from the colony image with the Lot No. of 3241 of the factory 3A and the colony image with the Lot No. of 8573 of the factory 3B, there is no problem with the inspection quality, for example, the method of culturing bacteria or the method of storing the petri dishes. This is because, when, for example, there is a problem in that the procedure of dilution was imperfect in the course of the culture, a wrong temperature was used to store a petri dish, or bacteria irrelevant to the inspection item was erroneously mixed, the colony images indicating that the number of colonies, the distribution mode of colonies, and the magnitude of the granularity of the colonies are significantly different from the two images described above are displayed. Furthermore, because there is no problem with the inspection quality between the most dissimilar colony images, also for the other colony images other than the two colony images described above, there is no big difference in the number of colonies, the distribution mode of the colonies, and the magnitude of the granularity of the colonies between the colony image with the Lot No. of 3241 of the factory 3A and the colony image with the Lot No. of 8573 of the factory 3B and thus it can be assumed that there is no problem with the overall inspection quality of the colony images in which the conditions are specified. Accordingly, it is also possible to omit the checking of the other colony images other than the two colony images displayed on the inspection quality investigation screen 300. Consequently, the number of checks of the colony images can be reduced when the inspection quality is investigated.

In this way, if there is no problem with the inspection quality, the person involved in the quality management department in the head office 5 presses and operates an OK button 310 on the inspection quality investigation screen 300. In this case, an OK log indicating that there is no problem with the inspection quality is created. In the OK log, it is possible to include the specification of the condition that is received by the receiving unit 14. For example, in the example illustrated in FIG. 7, it is possible to create the OK log in which the investigation result "OK" is associated with the investigation period of time "April", the investigation foodstuff "foodstuff A", and the investigation inspection item "general viable bacteria".

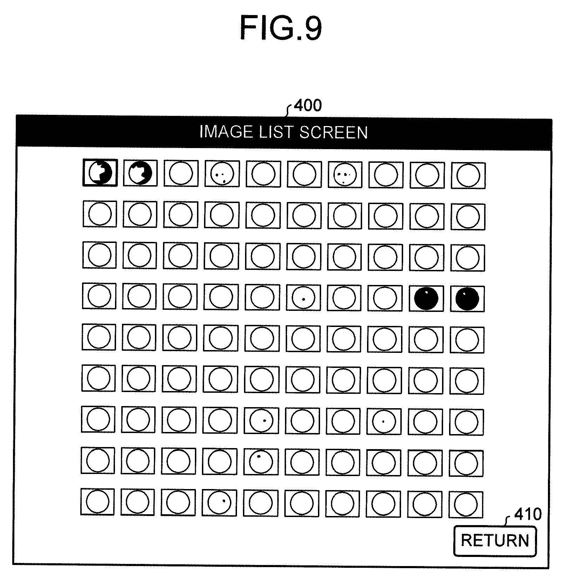

For example, if an inspection quality investigation screen 340 illustrated in FIG. 8 is displayed, as a pair of the most dissimilar colony images from among a lot of colony images, the images that have a big difference in the number of colonies, the distribution mode of the colonies, the magnitude of the granularity of the colonies are displayed side by side. Namely, the person involved in the quality management department in the head office 5 can determine that, for the specimen with the Lot No. of 3241 in the factory 3A between the two images, general viable bacteria is appropriately cultured and the hygiene inspection is appropriately performed, whereas the person involved can determine that, for the specimen with the Lot No. of 1111 of the factory 3C, there is a problem with the inspection quality, for example, the way of culturing bacteria or a method of storing the petri dish. In this way, if there is a problem with at least one of the two colony images, it is possible to presume that, for the other colony images other than the two colony images described above, the inspection quality may also possibly be abnormal. In this case, the person involved in the quality management department in the head office 5 presses and operates a side-by-side display button 320 on the inspection quality investigation screen 340, whereby the person involved can call an image list screen 400 illustrated in FIG. 9.

FIG. 9 is a schematic diagram illustrating an example of an image list screen. As illustrated in FIG. 9, on the image list screen 400, the list of the colony images acquired by the acquiring unit 15 is displayed in thumbnail. Consequently, the person involved in the quality management department in the head office 5 can visually check the colony image that has a problem with the inspection quality from among the colony images that are matched with the condition specified. Furthermore, by placing a mouse pointer over an image, the property information (the ship source or the like) related to the subject image can be displayed as a pop-up display. Furthermore, if a return button 410 on the image list screen 400 is pressed and operated, the display can be changed to the inspection quality investigation screen 340. Furthermore, here, a description has been given of a case in which, when the side-by-side display button 320 on the inspection quality investigation screen 340 is pressed and operated, the thumbnail displayed is performed; however, if the side-by-side display button 320 on the inspection quality investigation screen 300 is pressed and operated, the thumbnail display is similarly performed.

In this way, if there is a problem with the inspection quality, the person involved in the quality management department in the head office 5 presses and operates a contact-inspection-room button 330 on the inspection quality investigation screen 340. In this case, the person involved in the quality management department inputs the comment indicating that there is a problem with the inspection quality and sends an instruction to the inspection department by a mail or the like and then, a Laboratory Accident (L.A.) log is created as the inspection result. In the L.A. log, the specification of the condition received by the receiving unit 14 can be included. For example, in the example illustrated in FIG. 8, it is possible to create the L.A. log in which the inspection quality result "L.A." is associated with the inspection date "April xx", the inspection location "inspection room A", the inspection contact person "OO", the investigation foodstuff "foodstuff A", and the investigation inspection item "general viable bacteria". Furthermore, in the L.A. log, it is also possible to further associate the inspection information that is associated with the colony image that is selected by the person involved in the quality management department in the head office 5. For example, on the inspection quality investigation screen 340 or the image list screen 400, the person involved in the quality management department in the head office 5 is allowed to select the colony image that has a problem with the inspection quality. Then, the person involved in the quality management department in the head office 5 refers to the record of the inspection information associated with the colony image that was selected and operated. Furthermore, the specimen number of an imperfect hygiene inspection, the inspection date or the Lot No of the imperfect hygiene inspection, the ship source or a contact person performed the imperfect hygiene inspection, and the like may also be included in the L.A. log. Consequently, for example, it is possible to perform a hearing between the responsible person and the contact person in the inspection department in the factory 3. Thus, it is possible to conduct an investigation to determine the cause of the problem that has occurred in the hygiene inspection, take a countermeasure thereof, and instruct the responsible person or the contact person in the inspection department caused of a decrease in the inspection quality. Furthermore, in this example, a description has been given of a case in which the person involved in the quality management department investigates the inspection quality; however, the contact person in the inspection department may also manage the inspection quality by self-management. In this case, the contact person in the inspection department can browse the L.A. log or can input a comment of the handling with respect to the abnormality of the inspection quality to the history or a report, such as a monthly report.

The OK log and the L.A. log can be output to an arbitrary output destination. For example, the OK log and the L.A. log may also be output to the head office terminal 50, may also be stored in the storage device included in the colony image inspection device 10, or may also be output to the terminal device 30.

Furthermore, the registering unit 12, the receiving unit 14, the acquiring unit 15, the calculating unit 16, the selecting unit 17, and the output unit 18 described above can be implemented by a central processing unit (CPU), a micro processing unit (MPU), or the like executing a colony image inspection program. Furthermore, each of the functioning units described above can also be implemented by a hard wired logic, such as an application specific integrated circuit (ASIC), a field programmable gate array (FPGA), or the like.

Furthermore, for example a semiconductor memory device can be used for the image storing unit 13a and the inspection information storing unit 13b described above. For example, as an example of the semiconductor memory device, a video random access memory (VRAM), a random access memory (RAM), a read only memory (ROM), a flash memory, or the like may be included. Furthermore, instead of the internal memory, a storage device, such as a hard disk, an optical disk, or the like, may also be used.

### [Flow of a process]

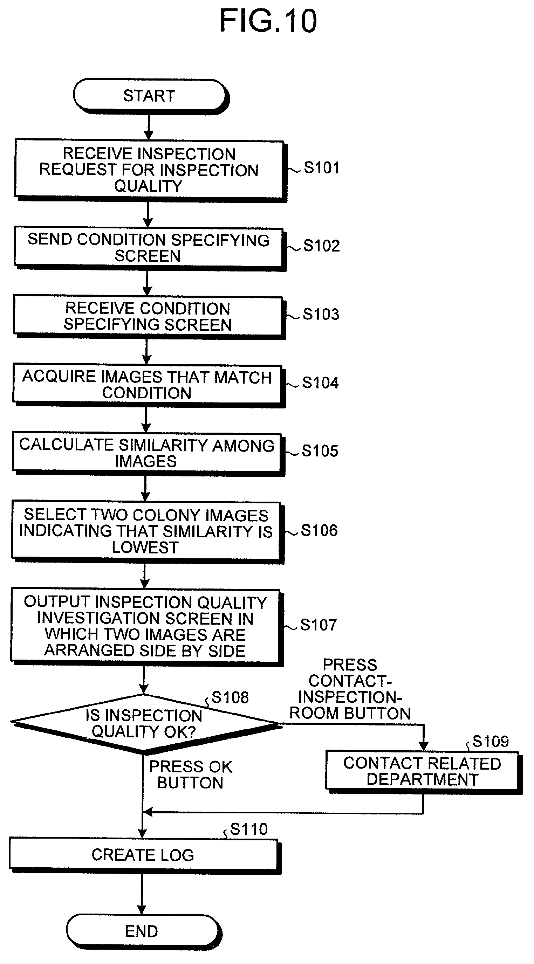

FIG. 10 is a flowchart illustrating the flow of a colony image inspection process according to the first embodiment. For this process, as an example, the process is started when an inspection request for the inspection quality is received from the head office terminal 50. For example, the process may also be started by selecting a function of an "image search", for example, the image search on a menu screen. Furthermore, the trigger of the process is not limited to a case in which a demand is received from the head office terminal 50. The process may also be started every time when a colony image is registered in the terminal device 30 or may also be automatically started periodically or irregularly, such as daily, weekly, monthly. Furthermore, if the inspection quality is managed, as the self-management, by the person involved in the inspection department in the factory 3, a demand may also be sent from the terminal device 30 in order to check the state of the inspection of the contact person and the responsible person themselves in the inspection room when, for example, the inspector has no confidence with the own investigation.

As illustrated in FIG. 10, when the receiving unit 14 receives an inspection request for the inspection quality from the head office terminal 50 (Step S101), the receiving unit 14 sends, to the head office terminal 50, the condition specifying screen on which conditions, such as the period of time, the foodstuff, the inspection item, the factory, the contact person, and the like (Step S102). Then, the receiving unit 14 receives the specified condition by receiving an input of specifying the conditions on the condition specifying screen that has been sent at Step S102 (Step S103).

Then, the acquiring unit 15 searches the inspection information storing unit 13b for the inspection information that is associated with the specified condition received at Step S103 and acquires, from the image storing unit 13a, the colony images that match the specified condition (Step S104).

Subsequently, the calculating unit 16 calculates the similarity among the three or more colony images that are acquired at Step S104 (Step S105). Then, from among the colony images acquired at Step S104, the selecting unit 17 selects the two colony images indicating that the similarity calculated at Step S105 is the most dissimilar (Step S106).

Then, the output unit 18 creates an inspection quality investigation screen in which the two colony images selected at Step S106 are vertically or horizontally arranged and sends the created inspection quality investigation screen to the head office terminal 50 (Step S107). At this point, if the side-by-side display button is pressed and operated on the inspection quality investigation screen, the output unit 18 can also send, to the head office terminal 50, the image list screen in which all of the colony images acquired at Step S104 are displayed in thumbnail.

Here, if the OK button is pressed and operated on the inspection quality investigation screen (Yes at Step S108), the output unit 18 creates an OK log indicating that there is no problem with the inspection quality (Step S110) and ends the process.

Furthermore, if the contact-inspection-room button is pressed and operated on the inspection quality investigation screen (No at Step S108), the output unit 18 performs a process (mail, display onto the screen indicated by the contact to the inspection system, or the like) of informing of the related inspection room (Step S109), creates an inspection quality investigation result log indicating that there is a problem with the inspection quality (Step S110), and ends the process.

### [Advantage of the first embodiment]

As described above, the colony image inspection device 10 according to the embodiment acquires three or more colony images of the same type bacterial colony captured in a petri dish and outputs a screen in which two images that are the most dissimilar from among the three or more colony images are arranged side by side. Consequently, for example, if it is possible to check that there is no problem with the inspection quality of the two colony images described above, also for the other colony images other than these, it is also possible to presume that no problem is present in the inspection quality. Thus, it is possible to omit the work of checking the other colony images other than the two colony images. Consequently, with the colony image inspection device 10 according to the embodiment, it is possible to reduce the trouble of investigating inspection quality.

### Second Embodiment

In the above explanation, a description has been given of the embodiments of the device according to the present invention; however, the present invention can be implemented with various kinds of embodiments other than the embodiments described above. Therefore, another embodiment included in the present invention will be described below.

### [Application example]

In the first embodiment described above, a description has been given of a case in which the two colony images that are the most dissimilar with each other are arranged side by side; however, from among the colony images, it is also possible to arrange two colony images, side by side, that are substantially similar in a degree in which both the colony images can be recognized as substantially the same images. For example, it is assumed of a case of using the similarity that has the upper limit of 1 and that indicates that both the colony images are more similar as the similarity is closer to 1. In this case, the colony images with the similarity of a predetermined threshold, for example, equal to or greater than 0.95, are arranged side by side. By doing so, it is possible to detect a state, such as a case in which the colony image that is used for the same hygiene inspection is erroneously registered or a case in which the colony image that is used for the same hygiene inspection is registered several times in order to forge the quality of a hygiene inspection.

### [Another applied case]

In the first embodiment described above, a description has been given of a case in which the person involved in the quality management department in the head office 5 investigates the inspection quality as a part of the quality management; however, in order to improve the skill of the inspector belonging to the inspection department in the factory 3, it may also possible to allow the responsible person in the inspection department in the factory 3 to investigate the inspection quality.

### [Separation and integration]

The components of each unit illustrated in the drawings are not always physically configured as illustrated in the drawings. In other words, the specific shape of a separate or integrated device is not limited to the drawings. Specifically, all or part of the device can be configured by functionally or physically separating or integrating any of the units depending on various loads or use conditions. For example, the receiving unit 14, the acquiring unit 15, the calculating unit 16, the selecting unit 17, or the output unit 18 may also be connected as an external device of the colony image inspection device 10 via a network. Furthermore, the receiving unit 14, the acquiring unit 15, the calculating unit 16, the selecting unit 17, or the output unit 18 are included by another device , are connected via the network, and cooperate with each other, whereby implementing the function performed by the colony image inspection device 10 described above. By doing so, even if a skilled person is located in a remote location, the inspection quality can be maintained constant.

### [Colony image inspection program]

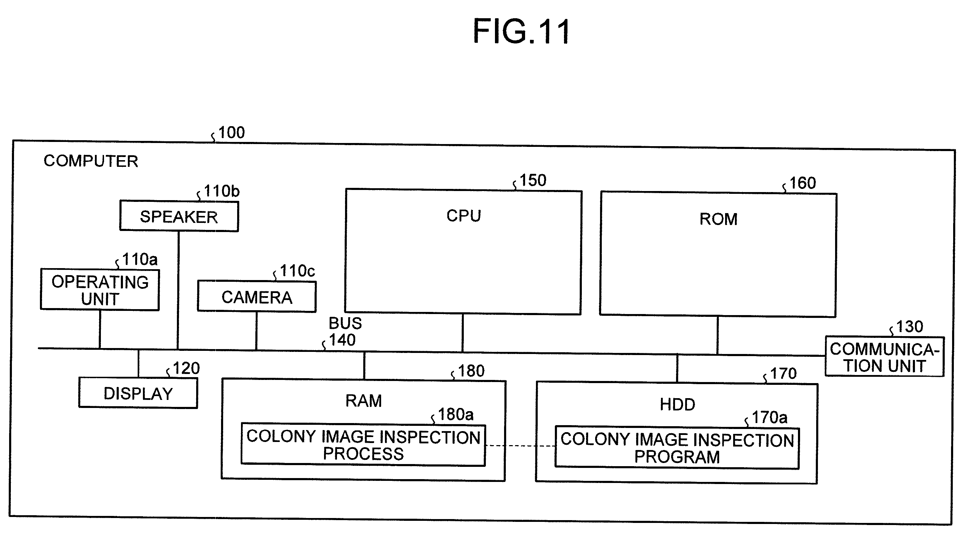

The various processes described in the above embodiments may also be implemented by a program prepared in advance and executed by a computer, such as a personal computer or a workstation. Accordingly, in the following, an example of a computer that executes a colony image inspection program having the same function as that performed in the embodiments described above will be described with reference to FIG. 11.

FIG. 11 is a schematic diagram illustrating an example of a computer that executes a colony image inspection program according to each of the first embodiment and a second embodiment. As illustrated in FIG. 11, a computer 100 includes an operating unit 110a, a speaker 110b, a camera 110c, a display 120, and a communication unit 130. Furthermore, the computer 100 includes a CPU 150, a ROM 160, an HDD 170, and a RAM 180. Each of the units 110 to 180 is connected via a bus 140. Furthermore, the camera 110c described above may also be connected by a USB or wirelessly connected as imaging equipment or.

As illustrated in FIG. 11, the HDD 170 stores therein, in advance, a colony image inspection program 170a having the same function as that performed by the receiving unit 14, the acquiring unit 15, the calculating unit 16, the selecting unit 17, and the output unit 18 described in the first embodiment. Similarly to each of the units, i.e., the receiving unit 14, the acquiring unit 15, the calculating unit 16, the selecting unit 17, and the output unit 18, that are illustrated in FIG. 2, the colony image inspection program 170a may also be integrated or separated. Specifically, not all pieces of the data need to be always stored in the HDD 170 as long as only the data needed for a process is stored in the HDD 170.

Then, the CPU 150 reads the colony image inspection program 170a from the HDD 170 and loads the program in the RAM 180. Consequently, as illustrated in FIG. 11, the colony image inspection program 170a functions as a colony image inspection process 180a. The colony image inspection process 180a loads, in an area of the RAM 180 allocated to various kinds of data that is read from the HDD 170 and executes various kinds of processes on the basis of the loaded various kinds of data. Furthermore, the colony image inspection process 180a includes the processes performed by the receiving unit 14, the acquiring unit 15, the calculating unit 16, the selecting unit 17, and the output unit 18 illustrated in FIG. 2, for example, the process illustrated in FIG. 10. Furthermore, for each of the processing units virtually implemented in the CPU 150, not all of the processing units are needed to be always operated in the CPU 150 as long as only the processing unit need to be operated is virtually implemented.

Furthermore, the colony image inspection program 170a does not always need to be initially stored in the HDD 170 or the ROM 160. For example, each program may be stored in a "portable physical medium", such as a flexible disk (FD), a CD-ROM, a DVD disk, a magneto-optic disk, a IC card, or the like, that is to be inserted into the computer 100. Then, the computer 100 may also read and execute the program from the portable physical medium. Furthermore, the program may be stored in another computer, a server device, or the like that is connected to the computer 100 through a public circuit, the Internet, a LAN, a WAN, or the like and the computer 100 may obtain the program from the other computer or the server device and execute the program.

### [Explanation of Reference]

- 1: quality management system
- 3A, 3B, 3C: factory
- 5: head office
- 7: network
- 10: colony image inspection device
- 11: communication I/F unit
- 12: registering unit
- 13a: image storing unit
- 13b: inspection information storing unit
- 14: receiving unit
- 15: acquiring unit
- 16: calculating unit
- 17: selecting unit
- 18: output unit
- 30A, 30B, 30C: terminal device
- 50: head office terminal

## Claims

1. A colony image inspection program comprising:
acquiring each of captured images of three or more petri dishes in each of which the same type of bacterial colony is cultured to be inspected;
calculating a similarity among the acquired three or more images;
selecting two images that indicate that the similarity is the lowest from among the three or more images; and
outputting the selected two images as images that are arranged side by side.

2. The colony image inspection program according to claim 1, wherein
information on a location in which the bacterial colony captured in each of the images is cultured or information on a contact person who cultured the bacterial colony is associated with each of the three or more images, and
the colony image inspection program further comprising receiving a specified input of information that indicates a location or a contact person, wherein
the acquiring includes acquiring the image that is associated with the same information on the location or the contact person as the received information that indicates the location or the contact person.

3. The colony image inspection program according to claim 1, or 2, wherein
information on a date on which each of the images are captured is associated with each of the three or more images, and
the colony image inspection program further comprising receiving a specified input of information that indicates a date, wherein
the acquiring includes acquiring the image that is associated with the same information on the date as the received information that indicates the date.

4. A colony image inspection method comprising:
acquiring each of captured images of three or more petri dishes in each of which the same type of bacterial colony is cultured to be inspected;
calculating a similarity among the acquired three or more images;
selecting two images that indicate that the similarity is the lowest from among the three or more images; and
outputting the selected two images as images that are arranged side by side.

5. The colony image inspection method according to claim 4, wherein
information on a location in which the bacterial colony captured in each of the images is cultured or information on a contact person who cultured the bacterial colony is associated with each of the three or more images, and
further comprising receiving, performed by the computer, a specified input of information that indicates a location or a contact person, wherein
the acquiring includes acquiring the image that is associated with the same information on the location or the contact person as the received information that indicates the location or the contact person.

6. The colony image inspection method according to claim 4 or 5, wherein
information on a date on which each of the images are captured is associated with each of the three or more images, and
further comprising receiving, performed by the computer, a specified input of information that indicates a date, and
the acquiring includes acquiring the image that is associated with the same information on the date as the received information that indicates the date.

7. A colony image inspection device comprising:
an acquiring unit that acquires each of captured images of three or more petri dishes in each of which the same type of bacterial colony is cultured to be inspected;
a calculating unit that calculates a similarity among the three or more images;
a selecting unit that selects two images that indicate that the similarity is the lowest from among the acquired three or more images; and
an output unit that outputs the selected two images as images that are arranged side by side.

8. The colony image inspection device according to claim 7, wherein
information on a location in which the bacterial colony captured in each of the images is cultured or information on a contact person who cultured the bacterial colony is associated with each of the three or more images, and
the colony image inspection device further comprising a receiving unit that receives a specified input of information that indicates a location or a contact person, wherein
the acquiring unit acquires the image that is associated with the same information on the location or the contact person as the received information that indicates the location or the contact person.

9. The colony image inspection device according to claim 7 or 8, wherein
information on a date on which each of the images are captured is associated with each of the three or more images, and
the colony image inspection device further comprising a receiving unit that receives a specified input of information that indicates a date, wherein
the acquiring unit acquires the image that is associated with the same information on the date as the received information that indicates the date.
